(19) Europäisches Patentamt
European Patent Office
Office européen des brevets

(11) **EP 3 545 843 A1**

(12) **EUROPEAN PATENT APPLICATION**

(43) Date of publication:
**02.10.2019 Bulletin 2019/40**

(51) Int Cl.:
**A61B 6/00** (2006.01)  **G02B 5/18** (2006.01)

(21) Application number: **18163965.9**

(22) Date of filing: **26.03.2018**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicant: **AGFA NV**
**2640 Mortsel (BE)**

(72) Inventors:
• **NEBOSIS, Rainer**
  **81539 München (DE)**
• **LEBLANS, Paul**
  **81539 München (DE)**

(54) **PCI VARIABLE PRE-SAMPLE MASK**

(57)    This invention is related to a grating assembly for use as a coded aperture or pre-sample mask in phase contrast imaging. The aperture size of the grating assembly is adjustable so that the imaging system may be optimized towards its intended imaging goal, being it beam deflection (preferential to obtain a phase image) or scatter (preferential for dark field image).

Fig. 2

EP 3 545 843 A1

## Description

## Technical Field

This invention is related to phase contrast imaging. Reference is made in the embodiments to particular examples in medical imaging.

## Background of the invention

Phase-contrast X-ray imaging (PCI) is a general term for different technical methods that use information concerning changes in the phase of an X-ray beam that passes through an object in order to create its images. Standard X-ray imaging techniques like radiography or computed tomography (CT) rely on a decrease of the X-ray beam's intensity (attenuation) when traversing the sample, which can be measured directly with the assistance of an X-ray detector. In PCI however, the beam's phase shift and scattering caused by the sample is not measured directly, but is transformed into variations in intensity, which then can be recorded by the detector.

When applied to samples that consist of atoms with low atomic number Z, PCI is more sensitive to density variations in the sample than conventional transmission-based X-ray imaging. This leads to images with improved soft tissue contrast, hence the particular interest for this technique in medical imaging.

Conventional X-ray imaging uses the drop in intensity through attenuation caused by an object in the X-ray beam and the radiation is treated as rays like in geometrical optics. But when X-rays pass through an object, not only their amplitude but their phase and propagation direction is altered as well. When applying the values of typical human tissue, the phase-shift of an X-ray beam propagating through tissue is up to three orders of magnitude larger than the loss in intensity caused by the absorption by the same tissue, thus making PCI more sensitive to density variations in soft tissue than absorption imaging.

A further advantage of PCI in medical diagnosis is that, because the phase contrast image formation is not intrinsically linked to the absorption of X-rays in the sample, the absorbed dose can potentially be reduced by using higher X-ray energies potentially saving the patient from substantial dose depositions during X-ray image acquisition.

Phase differences in X-ray beams cannot be directly measured by an X-ray detector, but require special interferometry techniques using diffraction gratings or an in-coherent setup with a plurality of apertures to produce the measurable effects at the X-ray detector. These measurable effects are small variations in the direction and width of X-ray beamlets obtained e.g. by means of a grating in between an X-ray source and a subject under examination, said variations being detected by means of an assembly of a detector mask placed before an X-ray detector.

Basically two approaches exist to produce said measurable effects:

1. An interferometric approach which uses a partially coherent source of radiation referred to as Talbot-Lau interferometry.
2. A completely in-coherent approach with repeated edge illumination referred to as Coded Aperture Phase Contrast Imaging.

The present invention is applicable to the second approach only, it will be described further on with reference to the Coded Aperture Phase Contrast Imaging technique.

The present invention is not limited to this embodiment. The principle of coded aperture phase contrast imaging (PCI) has been described in US 7, 869, 567.

X-rays emitted from an X-ray source are shaped into individual beamlets by means of selective absorption by a so-called "pre-sample mask" or "coded aperture" (which is an absorbing grating). The pre-sample mask allows only narrow portions of the X-ray beam to pass through so-called apertures of identical width. The pre-sample mask traditionally has a fixed geometry that is adapted to an analyser grating that in its turn is fitted to the geometry of the used X-ray detector pixel grid dimensions while taking into account the distances between the X-ray source, pre-sample mask and X-ray detector.

These narrow beamlets pass through a sample or object and arrive at individual pixels of the X-ray detector through said analyser grating. The individual X-ray beamlets are arranged to hit the pixel edge of individual rows of pixels or individual columns of pixels or individual pixels. Small deviations in the individual beamlets cause a significant increase or decrease in the signal hitting the exposed area of the pixel resulting in a significant phase contrast or dark field signal.

As suggested above, there exist two types of measurable effects caused by said aforementioned incidence of X-ray beamlets on an object and causing said small deviations. The first effect is the pure deflection of the X-ray beamlets influenced by the shape of the object or large structures inside the object (large in this context means some 100 $\mu$m in diameter) and giving rise to the "phase image". Secondly, scattering of the X-ray beamlets passing through an object and influenced by the pathlength of the beam through the object -and thus by the size and internal small structures (small in this context means below 100 $\mu$m in diameter) of the object- giving rise to the "scatter image" or "dark field image".

When using a standard X-ray tube, beam deflection also leads to virtual broadening of the beamlets, that is similar to beam scattering but with a smaller effect. This is caused by the polychromatic X-ray source which generates X-ray quanta with different energies. The refraction decrement of an object is dependent on the energy of the quanta. Thus using a polychromatic X-ray source (such as a conventional X-ray source used in medical applications) will lead to a variety of deflection angles due to the energy dependent refraction decrement. This effect virtually broadens the beamlets, and more specifically the intensity profile of the beamlet.

The width of a beamlet is at the same time also a measure for the scatter caused by the X-ray incidence on the object. More scatter causes a wider intensity profile. Beam deflection thus contributes to the measured differences and therefore lead to artefacts in the scatter image. Theoretically, an object which is not scattering but only deflects the beamlets would give rise to a non-zero (artificial) dark field image.

In the art, a compromise needed to be made for the choice of the exact aperture sizes of the pre-sample mask. As explained above, this choice significantly influences the sensitivity of the system for beam deflection (preferential to obtain a phase image) or scatter (preferential for dark field image). This choice was imposed by the fixed geometry of the pre-sample mask designs, and by the mechanical constraints to make such pre-sample mask. In most cases, a compromise was found between optimizing for phase and scatter imaging simultaneously.

A second aspect that determined the choice of the pre-sample mask geometry and design were the constraints of the production process of such a pre-sample mask requiring very small apertures. Typically, applications require mask sizes close to the size of the object to be imaged. In other words, the pre-sample mask should approximate the objects size in order to be able to provide a sufficient field of view. At the same time should the pre-sample mask have very narrow apertures of which the projections onto the used X-ray image detector approximate the pixel sizes of the detector itself. Producing relatively large masks with dimensions larger then 20cm x 20cm becomes difficult and very expensive, especially when aperture sizes of less than $100\mu m$ and grating pitches of less than $200\mu m$ are required. In a typical PCI setup, the dimensions of the apertures are in the range of $20\mu m$.

**Summary of invention**

The present invention provides a grating assembly comprising two parallel grating elements for use as a pre-sample mask or coded aperture in a phase contrast X-ray imaging system. The grating assembly comprises a first grating element comprising a multitude of parallel linear slits between absorbers with an aperture size $a_1$ and pitch $p_1$, a second grating element comprising a multitude of parallel linear slits between absorbers with an aperture size $a_2$ and pitch $p_2$, said grating elements being arranged such that the respective parallel linear slits of said first and second grating elements are arranged in parallel with each other, a holder keeping said first and second grating elements substantially parallel at a distance $Z_{G1G2}$ from each other, as set out in Claim 1. The grating assembly may further comprise an actuator to effectuate a linear parallel movement of said first grating element relative to said second grating element, said movement being perpendicular to the direction of the said parallel slits. It also provides for a phase contrast X-ray imaging system comprising said grating assembly.

In the context of this invention, a grating element is an element, that is preferably made from a sheet material that absorbs X-rays and that absorbs a part of the X-ray intensity at regular intervals due to its grid structure (hence the "coded" aperture). The grating element is typically embodied as a rectangular sheet of material comprising said grating. The grid structure or "grating" is preferably conceived as a repeating set of linear slits cut into the sheet of absorbing material. The slits are obtained by removing material from the sheet of absorbing material in a linear shape, and by cutting through its entire thickness. Other methods to produce a regular and repeating structure of absorbing and transmitting pairs may be applied. This may be achieved by techniques such as etching, mechanical cutting, laser cutting or alike. The choice of the thickness and material should be made based on its capacity to absorb at least 80% in the blocked area.

The linear slits are alternated with a repeating set of linear absorbers, which effectively are the very narrow linear remaining parts after cutting or etching out the slits. The linear absorbers block the X-ray intensity of the X-ray source, and constitute the blocked area of the grid structure or grating.

The size of the grating element depends on the imaging application (size of the object to be imaged), and is a function of the geometry of the X-ray system. In general, the size of the grating element is such that its X-ray shadow should cover the surface of the X-ray detector and the region of interest of the object entirely. The grating element is in principle positioned at some distance (typically 1 or 2m away) from an X-ray source with a relative small focus (in the range of 1mm or smaller) in order to obtain very narrow beamlets with small penumbra's. The distance of the grating to the detector is typically in the range of 0.5m to 1m.

In view of the above, the size of a typical grating element may in practice ideally be 20cm x 20cm or even larger. The pitch size of the grating element, i.e. the distance between two subsequent or neighbouring slits or absorbers should be adapted to the pixel size or pitch of the digital X-ray detector. The projected pitch size of the grating element should correspond to a multiple (1, 2, 3, ...) of the pitch size of the analyser grating that in its turn is fitted to the geometry of the used X-ray detector pixel grid used to capture the image in order to be suitable for phase contrast imaging. For systems without an analyser grating the pitch of the coded aperture shall be directly fitted to the pixel pitch and correspond to a multiple of the pixel size (2, 3, 4, ...).

The required pitch size for a grating element therefore is about $200\mu m$ or less and the aperture size is typically less than $100\mu m$ when using an analyser grating. In a typical PCI setup the dimensions of the aperture are in the range of 20μm, which makes the grating elements very difficult to produce, especially at the sizes put forward above.

In the context of this invention, a so-called grating assembly is presented to overcome a number of the problems known in the art with fixed grating elements (i.e. grating elements with fixed aperture and pitch size). A grating assembly is set of two grating elements that are combined into a single assembly, meaning that the two grating elements are in some way held together at a certain distance, but always in a parallel setup. The two grating elements, having each a high number of parallel linear slits, are aligned to each other so that their respective parallel linear slits are fully parallel.

The two grating elements are held in their position -parallel to each other-by means of a holder. The holder is a structure that holds each of the grating elements by its edges but still allows that either or both of the grating elements can slightly move in parallel to each other (and this in the direction perpendicular to the linear slits). The holder positions the grating assembly at the appropriate distance between the X-ray source and the digital X-ray detector. The holder determines the distance between the two parallel grating elements.

The grating assembly may further be foreseen with an actuator that allows to control the slight parallel movement in the holder of one of the grating elements with respect to one another, when actuated. The actuator may be a lever or a small micro-screw (or any other appropriate drive) system that allows to displace the first grating element in parallel to the second grating element, and this in a controlled way, meaning that the displacement caused by the actuator may be very small (a few μm's) but that the actuator still allows control over this small displacements in a controllable way. The actuator may be a mechanical system or motor driven.

The actuator allows both grating elements to be accurately aligned to each other in order to cause full, partial or no overlap of the linear slits in both grating elements. The choice over the amount of overlap of said linear slits allows accurate control over the aperture of the grating assembly, the largest aperture size of the assembly being limited by the smallest aperture size of either grating element, but the smallest aperture size not being limited at all.

Therefore the present invention is beneficial in that it allows control over the aperture size of the pre-sample mask or coded aperture in a PCI system. The variable aperture size has an important impact on the characteristics of the PCI system, whereas the fixed geometries were a compromise between optimizing for phase and scatter imaging simultaneously. The aperture size of the pre-sample mask may be varied between 0μm to more than 200μm, allowing the control over the sensitivity of the PCI system for beam deflection (phase image) or scatter (dark field image).

Another advantage of the invention is that the aperture sizes that are to be manufactured (for one grating element) may be relatively large, i.e. larger than 100μm. Such aperture sizes are much easier to manufacture in comparison with aperture sizes of 20μm or alike when using a single grating element as a pre-sample mask. The combination of two grating elements thus allows larger manufacturing aperture sizes. Traditional pre-sample masks (based on a single grating element) therefore were almost impossible to produce at larger sizes, which limited the field of view of the PCI system.

Another advantage of the application of a combination of two grating elements is that the aperture size may be adapted to requirements towards sensitivity of the PCI system.

Another advantage of the invention is that the distance between the X-ray source and the grating assembly may be adapted to adjust the measurement range (or magnification factor of the grating) to the object to be measured in order to avoid saturation of the measurement system (and thus to ensure correct measurement data). This can be done in two ways. Firstly only changing the projected pitch via magnification change and keeping the projected aperture size constant via adapted relative grating movement. Secondly change both the projected pitch and the aperture size. By changing the projected pitch size via magnification only and the projected aperture size via magnification and relative grating movement.

The optimization towards any of the two imaging types of PCI allows to obtain high image quality for both imaging modes with the same system. In short, a system that is optimized to detected scatter should not be too sensitive for beamlet deflection, and vice-versa. Therefore a variable aperture size is extremely useful to adapt and optimize a PCI system for high phase sensitivity or high scatter sensitivity.

Further advantages and embodiments of the present invention will become apparent from the following description and drawings. Specific examples and preferred embodiments are set out in the dependent claims.

**Brief description of drawings**

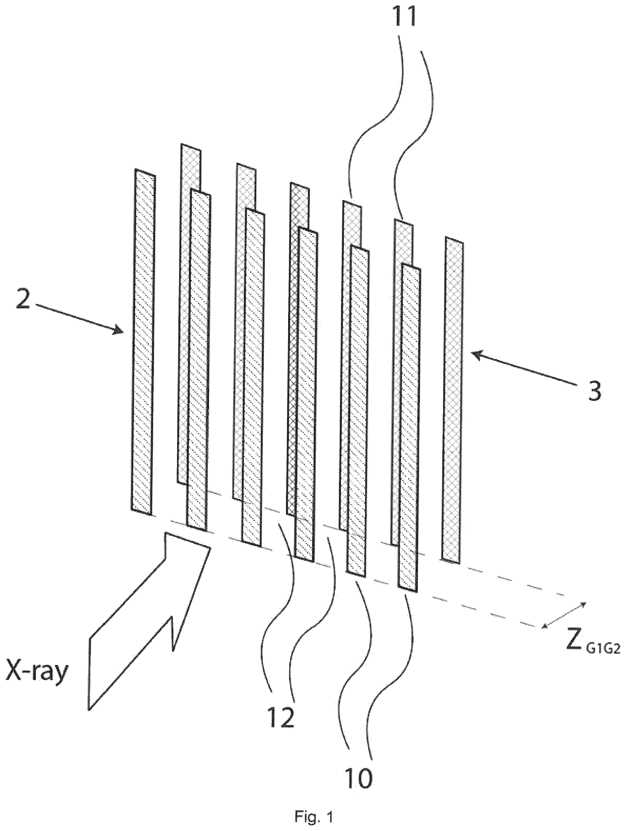

Fig. 1 gives a schematic overview of the principle of the two parallel grating elements (2)(3) which are oriented perpendicularly towards an X-ray source, and which comprise both a set of parallel absorbers (10)(11) respectively. The spaces between the parallel absorbers (10) on grating element (2) are the so-called linear slits or apertures (12). The grating elements (2) and (3) can move relatively to each other in the direction as indicated by the arrows, which is perpendicularly to the direction of the orientation of the linear slits of the grating elements. The indicated distance ($Z_{G1G2}$) is the distance between the two separate grating elements.

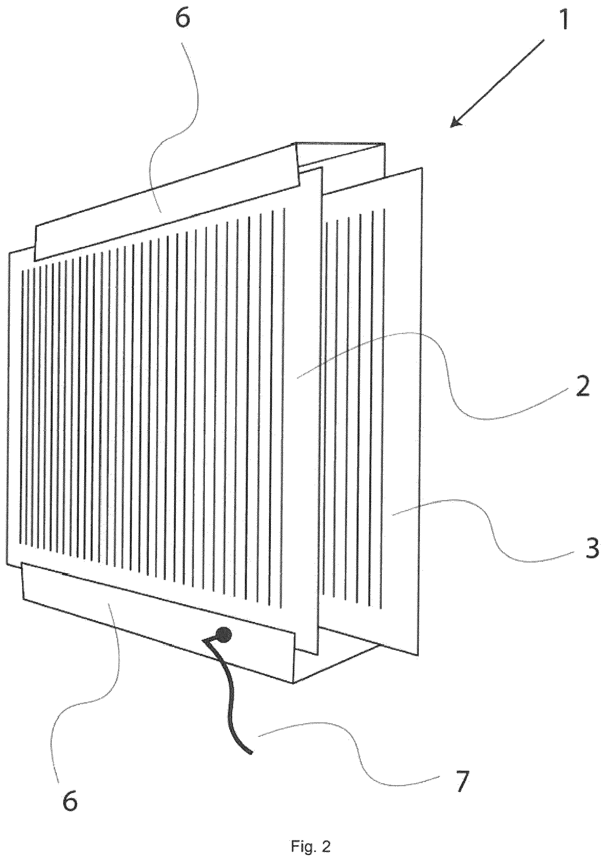

Fig. 2 gives a perspective view of a complete grating assembly wherein two grating elements (2) and (3) are visible as sheets of a material and comprising parallel linear slits. The grating elements are captured in a holder (6) which is visible at opposite sides of the grating elements. An actuator (7) is visible as a lever which allows the first grating element (2) to be displaced relative to a fixed grating element (3 in this case), and this in the direction perpendicular to the direction of the orientation of the linear slits of the grating elements.

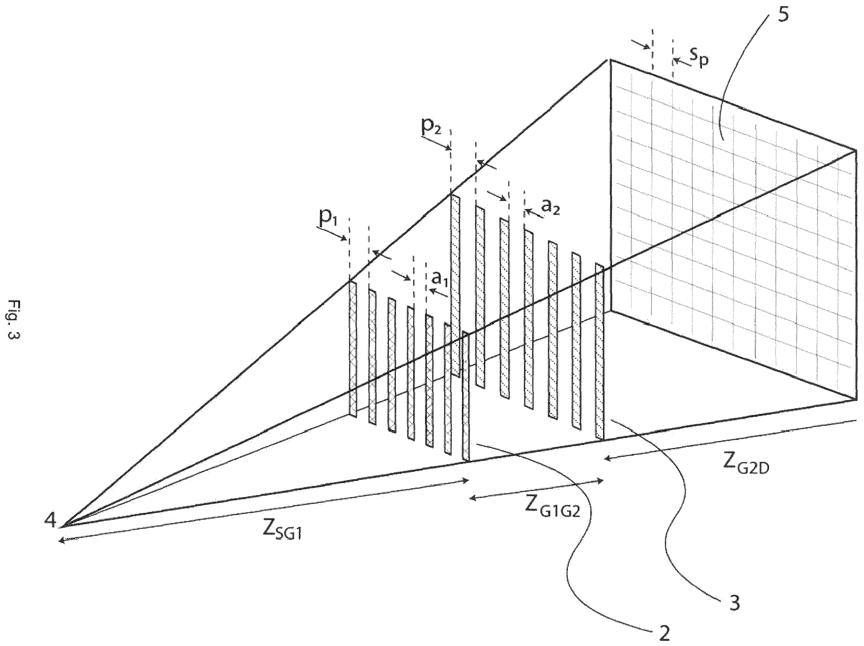

Fig. 3 gives a schematic overview of a complete PCI system, comprising an X-ray source (4), the two in parallel positioned grating elements (2) and (3), and the X-ray detector (5) which has a pixel pitch of $s_p$. The geometry of the system is determined by the distances indicated in the drawing; $Z_{SG1}$ is the distance between the X-ray source and the first grating element (2), $Z_{G1G2}$ is the (parallel) distance between both grating elements (2) and (3), and $Z_{G2D}$ is the distance between the second grating element and the X-ray detector.

Pitches $p_1$ and $p_2$ are adapted to the pixel pitch of the digital X-ray detector $s_p$, in such way that the projected pitch sizes $p_1$ and $p_2$ are multiples of the pixel pitch size $s_p$ of the X-ray detector.

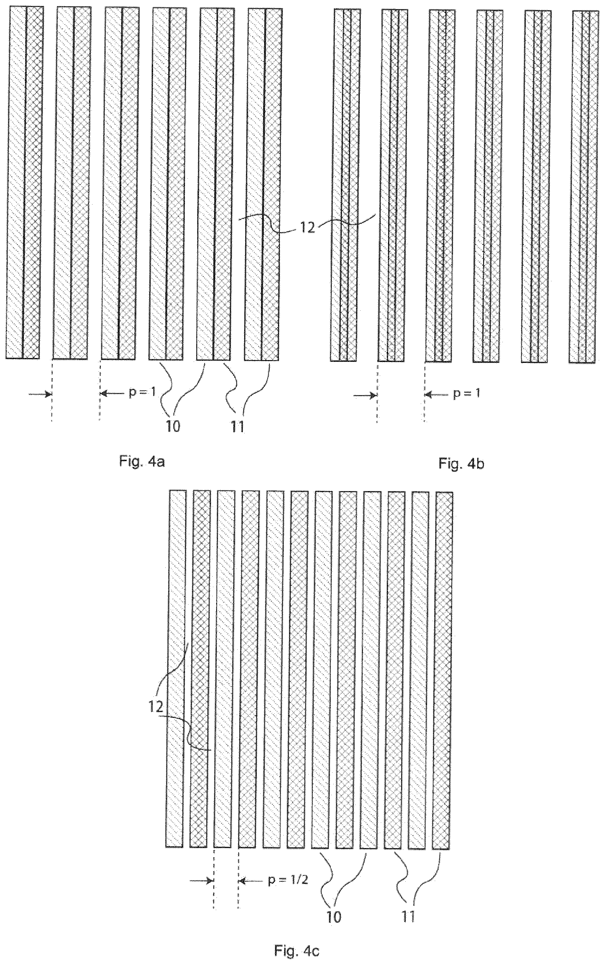

Fig. 4 illustrates the possibility of a dual grating configuration to obtain different pitch sizes by selecting the width of the absorbers smaller than the width of the apertures. Fig. 4a, b and c are showing different relative grating element positions of the same dual grating configurations.

Fig. 4a shows a schematic configuration of the two superimposed grating elements, of which the absorbers (10) of a first grating element (2) and the absorbers (11) of a second grating element (3) are indicated. The absorbers (10) and (11) are positioned such that they are adjacent to each other and thus forming one aperture (12). The pitch p of such configuration is indicated.

Fig. 4b shows the same configuration as in Fig. 4a, but with one of the grating elements slightly shifted in position so that the absorbers (10) of the first grating element (2) slightly overlap with the absorbers (11) of the second grating element (3). The result is a slightly larger aperture size (12), but an identical pitch p.

Fig. 4c shows the same configuration as in Fig. 4a and b, but wherein the absorbers (10) of the first grating element (2) are positioned right in the middle of two absorbers (11) of the second grating element (3), resulting in two apertures on either side of absorber (10), and resulting in a pitch which is half the size (or double frequency) of the pitch p of Fig. 4a and b.

## Description of embodiments

In the following detailed description, reference is made in sufficient detail to the above referenced drawings, allowing those skilled in the art to practice the embodiments explained below.

The grating assembly of the invention comprises two parallel grating elements, of which the first (2) is positioned at a distance $Z_{DG1}$ from the X-ray source (4), and a second grating element (3) further downstream the X-ray beam at a distance $Z_{G1G2}$ from the first grating element. The grating assembly is thus a structure that combines both grating elements into a single unit, kept together by a holder (6) which clamps both grating elements by their edges (avoiding that parts of the holder would be in the X-ray field of view) and keeps them at a well determined distance from each other. In a preferred embodiment, the holder keeps the grating elements (which are essentially sheets of X-ray absorbing material) almost in contact with each other (or on top of each other). In another embodiment, the grating elements are positioned in parallel at a selected certain distance $Z_{G1G2}$ from each other. This distance may be controlled by a second actuator (not shown in any of the drawings).

While the main function of the holder is to keep or clamp the grating elements into their position, it also allows the grating elements to make parallel movements perpendicular to the direction of the linear slits of the grating element. This parallel shift allows the relative positions of the absorbers of both grating elements to be controlled. This relative parallel movement allows the control and adjustment of the aperture size of the grating assembly. The means to control this parallel shifting movement is the first actuator (7), which may be embodied as any mechanism that allows fine control of the shifting movement, such as a lever, a gear set or alike.

The selection of the geometry of the two grating elements determine the range of aperture settings which may be eventually supported by the grating assembly. This will eventually determine the sensitivity of the PCI system for either beam deflection or scatter. It is clear that the geometry of the PCI system also determines the geometry of the two grating elements and their position in the PCI system itself. The requirements for the two grating elements for a specific PCI system geometry may be formulated as follows:

1- the absorption of the material of the grating element must be at least 80% in the blocking area,
2- transmission of the aperture must be at least 90%,
3- the pitch $p_1$ of the first grating element (2) must fulfil the following equation ($s_p$ is the X-ray detector pitch size):

$$p_1 = n.\frac{s_p.Z_{SG1}}{Z_{SG1} + Z_{G1G2} + Z_{G2D}};$$

where $n$ = 1; 2; 3; ...
4- the pitch $p_2$ of the second grating element (3) should fulfil the following equation:

$$p_2 = \frac{p_1 . (Z_{SG1} + Z_{SG2})}{Z_{SG1}}$$

Optionally, the aperture sizes for both grating elements should be around 50% of the respective pitch sizes.

In practice, the aperture size will be $\geq 100\mu m$, and the respective grating element pitch sizes will be > 1.5 x the respective aperture sizes.

The construction of the grating mechanism thus allows the adjustment of the aperture size, allowing optimization of the phase contrast image sensitivity.

Preferably the X-ray source for PCI has a typically small focus and is positioned at some distance from the grating assembly, which results in the production of narrow X-ray beamlets at the exit side of the grating element. The focal spot size may however also be used as an optimisation parameter; when adapted to a tuneable aperture size a more sophisticated adaption may be made to fulfil the needs of the application. This objective may be achieved by rotating the X-ray tube around an axis parallel to the grating aperture structures.

Another optimisation may be applied to the PCI system of our invention, namely the magnification factor that may be achieved using a grating with a certain pitch.

When exposing and imaging large objects, the produced scatter is higher compared to small objects. In order to avoid saturation and/or huge sensor pixel crosstalk (in the digital X-ray detector) under these high scatter conditions, the separation of the single beamlets should be increased in order to obtain a useable result. This can be achieved by either decreasing the aperture size and/or by increasing the magnification factor of the PCI system. The image magnification may be achieved in discrete magnification steps by changing the distance of the grating assembly to the X-ray source. The geometry (and thus positioning of the grating assembly) still needs to meet the criteria as stipulated above for determining the pitches of the grating elements, but in this case the variable aspect (the magnification) is determined by the factor n.

The assembly allows to change the projected pitch of the grating in discrete steps and simultaneously change the projected size of the aperture continuously and independent from the pitch change. Thus the width and the distance of the intensity profiles of single beamlets can be controlled independently. This allows a dynamic adaptation of the system performance to different applications i.e. phase or dark field imaging.

**Claims**

1. Grating assembly (1) comprising two parallel grating elements for use as a pre-sample mask or coded aperture in a phase contrast X-ray imaging system, comprising

   - a first grating element (2) comprising a multitude of parallel linear slits (12) between absorbers (10) with an aperture size $a_1$ and pitch $p_1$,
   - a second grating element (3) comprising a multitude of parallel linear slits between absorbers (11) with an aperture size $a_2$ and pitch $p_2$,
   - said grating elements being arranged such that the respective parallel linear slits of said first and second grating elements are arranged in parallel with each other,
   - a holder (6) keeping said first and second grating elements substantially parallel at a distance $Z_{G1G2}$ from each other.

2. Assembly of Claim 1, further comprising a first actuator (7) to effectuate a linear parallel movement of said first grating element (2) relative to said second grating element (3), said movement being perpendicular to the direction of the said parallel slits.

3. Assembly of any of the previous claims, further comprising a second actuator to effectuate a linear movement of said first grating element relative to said second grating element, said movement being perpendicular to their respective surfaces and altering distance $Z_{G1G2}$.

4. Assembly of any of the previous claims, wherein the first and second linear grating are stacked against each other.

5. Assembly of any of the previous claims, wherein the apertures are $\geq 100\mu m$.

6. Assembly of Claim 1, further comprising an actuator to effectuate a linear movement of said holder (6) perpendicular to the axis between said X-ray source and X-ray detector.

7. Phase contrast imaging X-ray system comprising,

   - an X-ray source (4),
   - a grating assembly (1) according to any of the previous claims wherein said first grating element is positioned at a distance $Z_{SG1}$ and perpendicularly from said source (4),
   - a digital X-ray detector (3) having a pixel size $s_p$ and positioned in parallel with said second grating element at a distance $Z_{G2D}$,
   - **characterized in that**:

$$p_1 = n . \frac{s_p . Z_{SG1}}{Z_{SG1} + Z_{G1G2} + Z_{G2D}};$$

*where n = 1; 2; 3; ... and*

$$p_2 = \frac{p_1 \cdot (Z_{SG1} + Z_{SG2})}{Z_{SG1}}$$

8. Phase contrast imaging X-ray system according to Claim 7, wherein said digital X-ray detector is fitted with a physical analyser grating, having a pitch size equal to the pixel size of said digital X-ray detector $s_p$.

9. Phase contrast imaging X-ray system according to Claim 7, wherein said digital X-ray detector is fitted with a physical or virtual analyser grating, having a pitch size = $n \cdot s_p$; $n \geq 2$.

10. Phase contrast imaging X-ray system according to Claim 7, 8 or 9, wherein said grating assembly is moveable along the axis between said X-ray source and X-ray detector.

11. Phase contrast imaging X-ray system according to Claim 7, 8, 9 or 10, wherein an object is positioned between said grating assembly and said digital X-ray detector.

12. Phase contrast imaging X-ray system according to Claims 7 to 11, wherein the focal spot size of the X-ray source is adapted by rotating the tube around an axis parallel to the parallel linear slits.

Fig. 1

Fig. 2

Fig. 3

p = 1

10    11

Fig. 4a

p = 1

Fig. 4b

12

p = 1/2

10    11

Fig. 4c

Europäisches
Patentamt

European
Patent Office

Office européen
des brevets

**EUROPEAN SEARCH REPORT**

Application Number

EP 18 16 3965

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2013/230135 A1 (HOSHINO YOSHIHIDE [JP] ET AL) 5 September 2013 (2013-09-05) | 1-6 | INV. A61B6/00 G02B5/18 |
| A | * paragraphs [0258], [0218] * | 7-12 | |
| A | WO 2012/098908 A1 (FUJIFILM CORP [JP]; TADA TAKUJI [JP]) 26 July 2012 (2012-07-26) * equation (49) * | 7-12 | |

TECHNICAL FIELDS
SEARCHED      (IPC)

A61B
G02B
G21K

The present search report has been drawn up for all claims

| Place of search | Date of completion of the search | Examiner |
|---|---|---|
| Munich | 14 September 2018 | Anscombe, Marcel |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons

& : member of the same patent family, corresponding
    document

EPO FORM 1503 03.82 (P04C01)

## ANNEX TO THE EUROPEAN SEARCH REPORT
## ON EUROPEAN PATENT APPLICATION NO.

EP 18 16 3965

14-09-2018

| Patent document cited in search report | | Publication date | Patent family member(s) | | Publication date |
|---|---|---|---|---|---|
| US 2013230135 | A1 | 05-09-2013 | JP<br>JP<br>US | 5857800 B2<br>2013180040 A<br>2013230135 A1 | 10-02-2016<br>12-09-2013<br>05-09-2013 |
| WO 2012098908 | A1 | 26-07-2012 | NONE | | |

EPO FORM P0459

For more details about this annex : see Official Journal of the European Patent Office, No. 12/82